(19) Europäisches Patentamt
European Patent Office
Office européen des brevets

(11) Veröffentlichungsnummer : **0 008 372**
**B1**

(12) # EUROPÄISCHE PATENTSCHRIFT

(45) Veröffentlichungstag der Patentschrift : **26.01.83**

(51) Int. Cl.³ : **C 07 D501/36**, A 61 K 31/545

(21) Anmeldenummer : **79102552.1**

(22) Anmeldetag : **19.07.79**

(54) **Leicht hydrolisierbare Cephalosporinester und -äther, deren Herstellung und pharmazeutische Präparate enthaltend solche Ester und Äther.**

(30) Priorität : **19.07.78 CH 7786/78**

(43) Veröffentlichungstag der Anmeldung : **05.03.80 Patentblatt 80/05**

(45) Bekanntmachung des Hinweises auf die Patenterteilung : **26.01.83 Patentblatt 83/04**

(84) Benannte Vertragsstaaten : **AT BE CH DE FR GB IT LU NL SE**

(56) Entgegenhaltungen :
**DE A 2 534 071**
**DE A 2 739 080**
**DE A 2 824 065**

**Die Akte enthält technische Angaben, die nach dem Eingang der Anmeldung eingereicht wurden und die nicht in dieser Patentschrift enthalten sind.**

(73) Patentinhaber : **F. HOFFMANN-LA ROCHE & CO. Aktiengesellschaft**
**CH-4002 Basel (CH)**

(72) Erfinder : **Montavon, Marc, Dr.**
**Realpstrasse 72**
**CH-4054 Basel (CH)**
Erfinder : **Reiner, Roland, Dr.**
**Rheinfelderstrasse 8**
**CH-4058 Basel (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

**EP 0 008 372 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Leicht hydrolysierbare Cephalosporinester und -äther, deren Herstellung und pharmazeutische Präparate enthaltend solche Ester und Äther

Die vorliegende Erfindung betrifft leicht hydrolysierbare Ester und Aether von Cephalosporinen der allgemeinen Formel

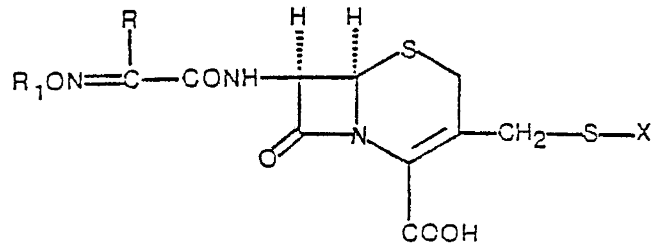

(I)

in der R Furyl, $R_1$ $C_{1-7}$ Alkyl und X eine Gruppe der Formeln

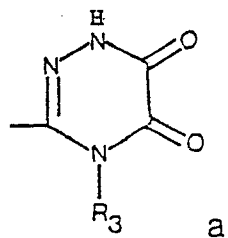

a

oder

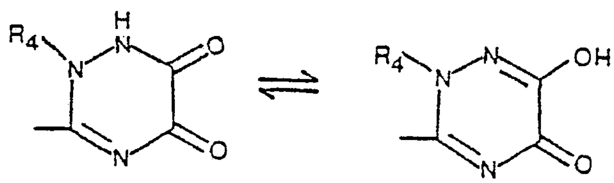

$c_1$ $c_2$

in denen $R_3$ und $R_4$ $C_{1-7}$ Alkyl darstellt, wobei « leicht hydrolysierbare Ester » Verbindungen der Formel I bedeuten, deren Carboxygruppe in Form einer leicht hydrolysierbaren Estergruppe vorliegt und « leicht hydrolysierbare Aether » Verbindungen der Formel I mit X = $c_2$ bedeuten, deren enolische OH-Gruppe in Form einer leicht hydrolysierbaren Aethergruppe vorliegt, sowie von Salzen dieser Verbindungen und von Hydraten dieser Ester, Aether und Salze.

Als leicht hydrolysierbare Ester der Verbindungen der Formel I sind Verbindungen der Formel I zu verstehen, deren Carboxygruppe in Form einer leicht hydrolysierbaren Estergruppe vorliegt. Beispiele solcher Ester sind die $C_{1-7}$-Alkyl-COO-$C_{1-7}$-alkylester, z.B. der Acetoxymethyl-, Pivaloyloxymethyl-, 1-Acetoxyäthyl- und 1-Pivaloyloxyäthylester ; die $C_{1-7}$-Alkoxy-COO-$C_{1-7}$-alkylester, z.B. der Methoxycarbonyloxymethyl-, 1-Aethoxycarbonyloxyäthyl- und 1-Isopropoxycarbonyloxyäthylester ; die Lactonylester, z.B. der Phthalidyl- und Thiophthalidylester ; die $C_{1-7}$-Alkoxy-$CH_2$-ester, z.B. der Methoxymethylester ; und die $C_{1-7}$-Alkyl-$CONHCH_2$-ester, z.B. der Acetamidomethylester. Auch andere Ester, z.B. die Benzyl- und Cyanmethylester, können brauchbar sein.

Als leicht hydrolysierbare Aether der Verbindungen der Formel I sind Verbindungen der Formel I mit X = $c_2$ zu verstehen, deren enolische OH-Gruppe in Form einer leicht hydrolysierbaren Aethergruppe vorliegt. Als Aethergruppen kommen die gleichen Gruppen in Betracht, wie sie oben bereits für die leicht hydrolysierbaren Estergruppen erwähnt wurden. Vertreter solcher Aether sind also beispielsweise die $C_{1-7}$-Alkyl-COO-$C_{1-7}$-alkyläther, z.B. der Acetoxymethyl-, Pivaloyloxymethyl-, 1-Acetoxyäthyl-, und 1-Pivaloyloxyäthyläther ; die $C_{1-7}$-Alkoxy-COO-$C_{1-7}$-alkyläther, z.B. der Methoxycarbonyloxymethyl-, 1-Aethoxycarbonyloxyäthyl- und 1-Isopropoxycarbonyloxyäthyläther ; die Lactonyläther, z.B. der Phthalidyl- und Thiophthalidyläther ; die $C_{1-7}$ Alkoxy-$CH_2$-äther, z.B. der Methoxymethyläther ; die $C_{1-7}$ Alkoxy-$CH_2$-äther, z.B. der Methoxymethyläther ; und die $C_{1-7}$-Alkyl-$CONHCH_2$-äther, z.B. der Acetamidomethyläther.

Beispiele von Salzen der erfindungsgemässen Ester bzw. Aether sind Salze mit Basen, z.B. Alkalimetallsalze, wie das Natrium- und Kaliumsalz ; das Ammoniumsalz ; Erdalkalimetallsalze, wie das Calciumsalz ; Salze mit organischen Basen, wie Salze mit Aminen, z.B. Salze mit N-Aethylpiperidin,

Procain, Dibenzylamin, N,N'-Dibenzyläthyläthylendiamin, Alkylaminen oder Dialkylaminen, sowie Salze mit Aminosäuren, wie z.B. Salze mit Arginin oder Lysin. Bedingung für die Bildung von Salzen mit Basen ist, dass mindestens eine der Carboxy- bzw. enolischen OH-Gruppen der Verbindung der Formel I frei vorliegt.

Die erfindungsgemässen Ester und Aether bilden auch Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Mono-arylsulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die erfindungsgemässen Produkte können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die in der Definition der Verbindungen der Formel I genannten $C_{1-7}$-Alkylgruppen sind entweder geradkettig oder verzweigt und können bis zu 7 Kohlenstoffatome enthalten, z.B. Methyl, Aethyl, n-Propyl, Isopropyl, n-Pentyl, n-Heptyl. $C_{1-7}$-Alkoxygruppen haben analoge Bedeutung. Halogen stellt alle vier Halogene dar, d.h. Fluor, Chlor, Brom und Jod ; bevorzugt sind Chlor und Brom.

Bevorzugte R-Gruppen sind Furyl, Thienyl und Phenyl, insbesondere Furyl. Als $R_1$ ist Methyl bevorzugt. Bevorzugte X-Gruppen sind die Gruppe d sowie die Gruppe a, worin einer der beiden Reste $R_2$ und $R_3$ Wasserstoff und der andere Methyl darstellt, ferner die Gruppen b und $c_1$ bzw. $c_2$ worin $R_4$ Methyl darstellt. Besonders bevorzugte X-Gruppen sind die 1,2,5,6-Tetrahydro-2-methyl-5,6-dioxo-as-triazin-3-yl-gruppe, deren entsprechende tautomere Form, die 2,5-Dihydro-6-hydroxy-2-methyl-5-oxo-as-triazin-3-ylgruppe (welche, wie oben erwähnt, an der Hydroxygruppe veräthert ist) und die 1,4,5,6-Tetrahydro-4-methyl-5,6-dioxo-astriazin-3-yl-gruppe.

Die Ester bzw. Aether der Verbindungen der Formel I sowie deren Salze und Hydrate können in der syn-isomeren Form

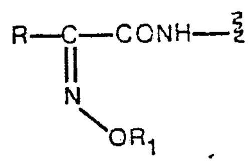

oder in der anti-isomeren Form

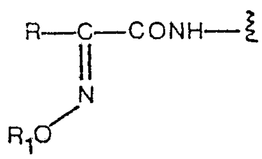

bzw. als Gemische dieser beiden Formen vorliegen. Bevorzugt ist die syn-isomere Form bzw. Gemische, in denen die synisomere Form überwiegt.

Bevorzugte Verbindungen sind :

Methylen-(6R, 7R)-7-[2-(2-furyl)-2-(methoxyimino)acetamido]-8-oxo-3-/[(1,4,5,6-tetrahydro-4-methyl-5,6-dioxo-astriazin-3-yl)thio]methyl/-5-thia-1-azabicyclo[4.2.0]oct-2-en-2-carboxylat-pivalat (Z-Isomer),

Methylen-(6R, 7R)-3-{/[2,5-dihydro-2-methyl-5-oxo-6-[(pivaloyloxy)methoxy]-as-triazin-3-yl]thio/methyl}-7-[2-(2-furyl)-2-(methoxyimino)acetamido]-8-oxo-5-thia-1-azabicyclo[4.2.0]oct-2-en-2-carboxylat-pivalat (Z-Isomer), und deren Salze sowie die entsprechenden Hydrate.

Die erfindungsgemässen Cephalosporinester und -äther werden erfindungsgemäss dadurch hergestellt, dass man

a) eine Carbonsäure bzw. ein Enol der Formel I einer entsprechenden Veresterung bzw. Verätherung unterwirft oder dass man

b) einen leicht hydrolysierbaren Ester bzw. Aether einer Carbonsäure der allgemeinen Formel

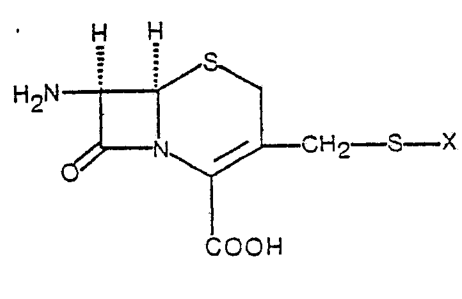

(II)

in der X die in Formel I gegebene Bedeutung hat, mit einer Säure der allgemeinen Formel

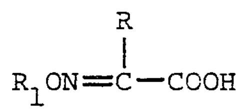

(III)

in der R und $R_1$ die in Formel I gegebene Bedeutung haben, oder mit einem reaktionsfähigen funktionellen Derivat dieser Säure umsetzt oder dass man

c) zur Herstellung eines leicht hydrolysierbaren Aethers einer Verbindung der Formel I eine Carbonsäure der allgemeinen Formel

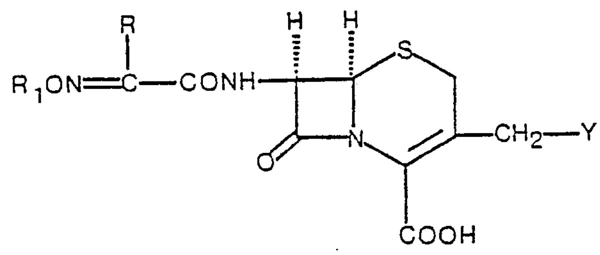

(IV)

in der R und $R_1$ die in Formel I gegebene Bedeutung haben und Y eine austretende Gruppe darstellt mit einem Thiol der allgemeinen Formel

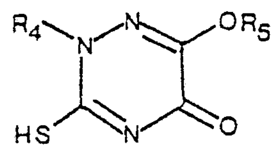

(V)

in der $R_4$ die in Formel I gegebene Bedeutung hat und $R_5$ eine leicht abspaltbare Aethergruppe darstellt, umsetzt, wonach man das Reaktionsprodukt ggfs. in ein Salz oder Hydrat bzw. ein Hydrat dieses Salzes überführt.

Zur Herstellung der leicht hydrolysierbaren Ester der Carbonsäuren der Formel I gemäss Variante a) wird eine Carbonsäure der Formel I vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Bei Vorhanden sein der Gruppe $c_2$ mit ihrer enolischen Funktion wird diese unter Bildung eines entsprechenden, leicht hydrolysierbaren Aethers veräthert. Vorzugsweise verwendet man dabei einen Ueberschuss an dem entsprechenden Halogenid. Die Veresterungs-/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40 °C.

Die leicht hydrolysierbaren Ester bzw. Aether der Carbonsäuren der Formel II, welche für Variante b) als Ausgangsverbindungen in Betracht kommen, werden in zu Variante a) ganz analoger Weise hergestellt.

Als reaktionsfähige funktionelle Derivate von Säuren der Formel III kommen z.B. Halogenide, d.h. Chloride, Bromide und Fluoride ; Azide ; Anhydride, insbesondere gemischte Anhydride mit stärkeren Säuren ; reaktionsfähige Ester, z.B. N-Hydroxysuccinimidester, und Amide, z.B. Imidazolide, in Betracht.

Die Umsetzung eines leicht hydrolysierbaren Esters bzw. Aethers einer Carbonsäure der Formel II mit einer Säure der Formel III oder einem reaktionsfähigen funktionellen Derivat davon gemäss Variante b) kann in an sich bekannter Weise durchgeführt werden. So kann man z.B. eine freie Säure der Formel III mit einem der erwähnten Ester bzw. Aether mittels eines Carbodiimids, wie Dicyclohexylcarbodiimid, in einem inerten Lösungsmittel, wie Essigester, Acetonitril, Dioxan, Chloroform, Methylenchlorid, Benzol oder Dimethylformamid kondensieren. Anstelle von Carbodiimiden lassen sich als kondensationsmittel auch Oxazoliumsalze, z.B. N-Aethyl-5-phenyl-isoxazolium-3'-sulfonat verwenden.

Nach einer weiteren Ausführungsform der Variante b) wird als Ausgangsmaterial ein Säurehalogenid, vorzugsweise das Chlorid einer Säure der Formel III, verwendet. Diese Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet ; es kann aber auch in einem aprotischen organischen Lösungsmittel, wie z.B. Dimethylformamid, Dimethylsulfoxid, oder Hexamethylphosphorsäuretriamid, gearbeitet werden.

Die Umsetzung eines leicht hydrolysierbaren Esters bzw. Aethers einer Verbindung der Formel II mit

einer Verbindung der Formel III oder einem reaktionsfähigen funktionellen Derivat davon kann zweckmässig bei Temperaturen zwischen etwa − 40 °C und Zimmertemperatur, beispielsweise bei etwa 0-10 °C, erfolgen.

Als austretende Gruppe Y einer Verbindung der Formel IV kommen beispielsweise Halogene, z.B. Chlor, Brom oder Jod, Acyloxyreste, z.B. $C_{1-7}$-Alkyl-CO-reste, wie Acetoxy, $C_{1-7}$-Alkyl- oder Arylsulfonyloxyreste, wie Mesyloxy oder Tosyloxy, oder der Azidorest in Frage.

Die Umsetzung einer Verbindung der Formel IV mit einem Thiol der Formel V gemäss Variante c) kann in an sich bekannter Weise, z.B. bei einer Temperatur zwischen etwa 40 und 80 °C, zweckmässig bei etwa 60 °C, in einem polaren Lösungsmittel, beispielsweise in einem Alkohol, wie z.B. in einem niederen Alkanol, wie Aethanol, Propanol und dgl., in Dimethylformamid oder Dimethylsulfoxid, vorzugsweise in Wasser oder in einer Pufferlösung mit einem pH von etwa 6 bis 7, vorzugsweise 6,5, durchgeführt werden.

Die als Ausgangsprodukte verwendeten, leicht hydrolysierbaren Ester bzw. Aether der 7-Aminoverbindungen der Formel II können ausgehend von einem leicht hydrolysierbaren Ester bzw. Aether einer Verbindung der Formel

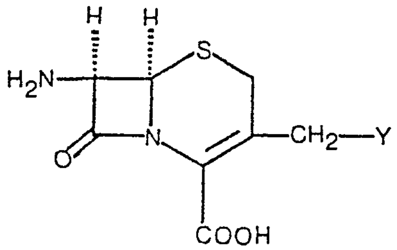

(VI)

in der Y eine austretende Gruppe darstellt, mit einem Thiol der Formel V hergestellt werden. Die Umsetzung kann unter den gleichen Bedingungen wie diejenige der Ausgangsverbindungen IV und V erfolgen.

Ein allenfalls erhaltenes syn/anti-Gemisch eines erfindungsgemässen Esters und Aethers kann in die entsprechenden syn- und anti-Formen in üblicher Weise getrennt werden, beispielsweise durch Umkristallisation oder durch chromatographische Methoden unter Verwendung eines geeigneten Lösungsmittels bzw. Lösungsmittelgemisches.

Die erfindungsgemässen Produkte sind antibiotisch, insbesondere bakterizid wirksam. Sie besitzen ein breites Wirkungsspektrum gegen Gram-positive und Gram-negative Mikroorganismen, einschliesslich β-Lactamase-bildende Staphylokokken und verschiedene β-Lactamase-bildende Gram-negative Bakterien, wie z.B. Haemophilus influenzae, Escherichia coli, Proteus- und Klebsiella-Spezies.

Die erfindungsgemässen Produkte können zur Behandlung und Prophylaxe von Infektionskrankheiten verwendet werden. Für den Erwachsenen kommt eine Tagesdosis von etwa 1 g bis etwa 4 g in Betracht. Die Produkte können sowohl oral als auch parenteral verabreicht werden.

Zum Nachweis der antimikrobiellen Wirksamkeit der erfindungsgemässen Verbindungen wurden folgende repräsentative Vertreter getestet:

Produkt A: Methylen-(6R, 7R)-7-[2-(2-furyl)-2-(methoxyimino)-acetamido]-8-oxo-3-/[(1,4,5,6-tetrahydro-4-methyl-5,6-dioxo-as-triazin-3-yl)thio]methyl/-5-thia-1-azabicyclo[4.2.0]oct-2-en-2-carboxylatpivalat (Z-Isomer);

Produkt B: Methylen-(6R, 7R)-3-{/[2,5-dihydro-2-methyl-5-oxo-6-[(pivaloyloxy)methoxy]-as-triazin-3-yl]thio/methyl}-7-[2-(2-furyl)-2-(methoxyimino)-acetamido]-8-oxo-5-thia-1-azabicyclo[4.2.0]oct-2-en-2-carboxylat-pivalat (Z-Isomer).

Aktivität in vivo

Gruppen von 5 Mäusen werden mit einer wässrigen Suspension von E. coli intraperitoneal infiziert. 1,2 1/2 und 4 Stunden nach der Infektion wird die Prüfsubstanz oral bzw. subcutan appliziert. Die Zahl der überlebenden Tiere wird am 4. Tag bestimmt. Es werden verschiedene Dosierungen appliziert, und durch Interpolation wird diejenige Dosis bestimmt, bei der 50 % der Versuchstiere überlebten ($CD_{50}$, mg/kg).

| Prüfsubstanz | A (oral) | A (subcutan) | B (oral) | B (subcutan) |
|---|---|---|---|---|
| $CD_{50}$, mg/kg | 0,05 | 0,02 | 0,03 | 0,02 |

Toxizität (Maus, 24-Stundenwerte)

| Prüfsubstanz | A | B |
|---|---|---|
| $LD_{50}$, mg/kg | | |
| p.o. | 2 500-5 000 | 1 250-2 500 |

Die erfindungsgemässen Produkte unterscheiden sich von bekannten Verbindungen aus der DOS 25 34 071 durch eine $C_{1-7}$-Alkoxyiminogruppe in Position 2 des 2-Furylacetamidosubstituenten in Stellung 7. Dieser Unterschied hat eine erhöhte antimikrobielle Wirkung zur Folge, insbesondere gegen Escherichia coli. Die erfindungsgemässen Produkte stellen demgemäss eine wertvolle Bereicherung des Arzneimittelschatzes dar.

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln ; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutisch wertvolle Stoffe enthalten.

## Beispiel 1

Methylen-(6R, 7R)-7-[2-(2-furyl)-2-(methoxyimino)acetamido]-8-oxo-3-/(1,4,5,6-tetrahydro-4-methyl-5,6-dioxo-as-triazin-3-yl)thio]methyl/-5-thia-1-azabicyclo[4.2.0]oct-2-en-2-carboxylat-pivalat (Z-Isomer) :

5,2 g (7R)-7-[2-(2-Furyl)-2-(methoxyimino)acetamido]-3-/[(1,4,5,6-tetrahydro-4-methyl-5,6-dioxo-as-triazin-3-yl)thio]methyl/-3-cephem-4-carbonsäure werden in 30 ml Dimethylformamid bei 0° mit 2,8 ml Triäthylamin und 5,3 g Jodmethylpivalat versetzt und 10 Minuten unter Stickstoffbegasung nachgerührt. Man verdünnt mit 250 ml Aethylacetat und extrahiert mit 5 %iger wässriger Natriumbicarbonatlösung und Wasser. Die Aethylacetat-Lösung wird mit Magnesiumsulfat getrocknet und eingedampft. Das erhaltene Oel wird in wenig Aethylacetat gelöst und mit tiefsiedendem Petroläther ausgefällt. Die abfiltrierte Substanz wird in Aethylacetat gelöst und mit Aethylacetat über Kieselgel chromatographiert. Die einheitlichen Fraktionen werden eingedampft und das erhaltene Oel wird aus Aethylacetat mit n-Hexan gefällt. Nach dem Abnutschen und Trocknen erhält man die reine Titelsubstanz :

$[\alpha]_D^{25} = -66{,}1°$ (c = 1 in Aethylacetat)
NMR : Spektrum in Uebereinstimmung mit der Struktur
Die Substanz enthält 0,3 Mol n-Hexan

| | | | | |
|---|---|---|---|---|
| Berechnet : | C 48,35 % | H 4,84 % | N 12,76 % | S 9,74 % |
| Gefunden : | C 48,17 % | H 5,05 % | N 12,39 % | S 9,60 % |

## Beispiel 2

Methylen-(6R, 7R)-3-{/[2,5-dihydro-2-methyl-5-oxo-6-[(pivaloyloxy)methoxy]-as-triazin-3-yl]thio/methyl}-7-[2-(2-furyl)-2-(methoxyimino)acetamido]-8-oxo-5-thia-1-azabicyclo[4.2.0]oct-2-en-2-carboxylat-pivalat (Z-Isomer).

5,7 g (Z)-(7R)-[2-(2-Furyl)-2-(methoxyimino)acetamido]-3-/[(2,5-dihydro-6-hydroxy-2-methyl-5-oxo-as-triazin-3-yl)thio]methyl/-3-cephem-4-carbonsäure-Dinatriumsalz werden in 30 ml Dimethylformamid auf 0° abgekült, unter Rühren mit 2,8 ml Triäthylamin und danach mit 5,3 g Jodmethylpivalat versetzt und 10 Minuten unter Stickstoff nachgerührt. Nach dem Verdünnen mit 250 ml Aethylacetat wird mit 5 %iger wässriger Natriumbicarbonatlösung und Wasser gewaschen, über Magnesiumsulfat getrocknet und bei 12 Torr. eingedampft. Das erhaltene Oel wird in wenig Aethylacetat gelöst und mit tiefsiedendem Petroläther ausgefällt. Die abgenutschte Substanz wird in Aethylacetat gelöst und über Kieselgel mit Aethylacetat chromatographiert. Man erhält aus den einheitlichen Fraktionen nach dem Eindampfen und Ausfällen aus Aethylacetat mit tiefsiedendem Petroläther die Titelsubstanz :

$[\alpha]_D^{25} = -200{,}2°$ (c = 1 in Aethylacetat)
Smp : ab 105°
NMR : Spektrum in Uebereinstimmung mit der Struktur

| | | | | |
|---|---|---|---|---|
| Berechnet : | C 49,59 % | H 5,10 % | N 11,19 % | S 8,54 % |
| Gefunden : | C 50,08 % | H 5,22 % | N 11,00 % | S 8,41 % |

## Beispiel 3

Herstellung von Trockenampullen für die parenterale Verabreichung :

Es wird in üblicher Weise ein Lyophilisat von 500 mg Methylen-(6R, 7R)-7-[2-(2-furyl)-2-(methoxyimino)acetamido]-8-oxo-3-/[(1,4,5,6-tetrahydro-4-methyl-5,6-dioxoas-triazin-3-yl)thiol]methyl/-5-thia-1-azabicyclo[4.2.0]octo-2-en-2-carboxylat-pivalat (Z-Isomer) hergestellt und in eine Ampulle abgefüllt.

## Beispiel 4

Es wird in üblicher Weise eine Gelatine-Steckkapsel folgenden Inhaltes hergestellt :

| | |
|---|---|
| Methylen (6R, 7R)-3-{/[2,5-dihydro-2-methyl-5-oxo-6-[(pivaloyloxy)methoxy]-as-triazin-3-yl}thio/methyl]-7-[2-(2-furyl)-2-methoxyimino)acetamido]-8-oxo-5-thia-1-azabicyclo[4.2.0]oct-2-en-2-carboxylat pivalat (Z-Isomer) | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

## Ansprüche

1. Leicht hydrolysierbare Ester und Aether von Cephalosporinen der allgemeinen Formel

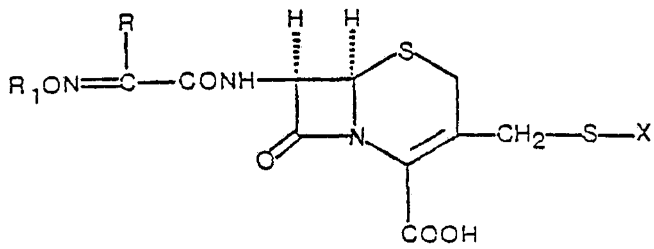

(I)

in der R Furyl, $R_1$ $C_{1-7}$ Alkyl und X eine Gruppe der Formeln

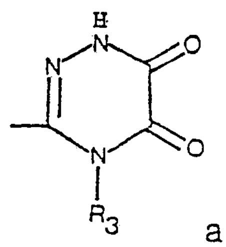

a

oder

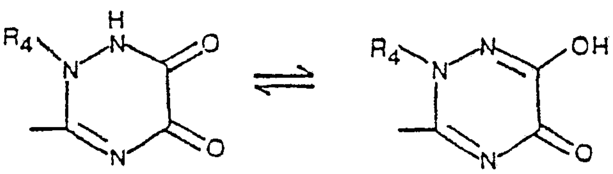

$c_1$ $c_2$

in denen $R_3$ und $R_4$ $C_{1-7}$ Alkyl darstellt, wobei « leicht hydrolysierbare Ester » Verbindungen der Formel I bedeuten, deren Carboxygruppe in Form einer leicht hydrolysierbaren Estergruppe vorliegt und « leicht hydrolysierbare Aether » Verbindungen der Formel I mit X = $c_2$ bedeuten, deren enolische OH-Gruppe in Form einer leicht hydrolysierbaren Aethergruppe vorliegt, sowie von Salzen dieser Verbindungen und von Hydraten dieser Ester, Aether und Salze.

2. Ester und Aether gemäss Anspruch 1, dadurch gekennzeichnet, dass sie $C_{1-7}$-Alkyl-COO-$c_{1-7}$-alkylester bzw. -äther darstellen.

3. Ester und Aether gemäss Anspruch 2, dadurch gekennzeichnet, dass sie Pivaloyloxymethylester bzw. -äther darstellen.

4. Ester und Aether von Cephalosporinen der Formel I gemäss einem der Ansprüche 1-3 dadurch gekennzeichnet, dass $R_1$ Methyl darstellt, sowie Salze dieser Verbindungen und Hydrate dieser Ester, Aether und Salze.

5. Ester und Aether von Cephalosporinen der Formel I gemäss einem der Ansprüche 1-4, dadurch gekennzeichnet, dass X die 1,2,5,6-Tetrahydro-2-methyl-5,6-dioxo-as-triazin-3-yl-bzw. die 2,5-Dihydro-6-hydroxy-2-methyl-5-oxo-as-triazin-3-yl-gruppe darstellt, sowie Salze dieser Verbindungen und Hydrate dieser Ester, Aether und Salze.

6. Ester und Aether von Cephalosporinen der Formel I gemäss einem der Ansprüche 1-5, dadurch

gekennzeichnet, dass X die 1,4,5,6-Tetrahydro-4-methyl-5,6-dioxo-as-triazin-3-yl-gruppe darstellt, sowie Salze dieser Verbindungen und Hydrate dieser Ester, Aether und Salze.

7. Methylen-(6R, 7R)-3-{/[2,5-dihydro-2-methyl-5-oxo-6[(pivaloyloxy)methoxy]-as-triazin-3-yl]thio/methyl}-7-[2-(2-furyl)-2-(methoxyimino)acetamido]-8-oxo-5-thia-1-azabicyclo[4.2.0]oct-2-en-2-carboxylat-pivalat (Z-Isomer), sowie Salze dieser Verbindung und Hydrate dieser Verbindung und von deren Salzen.

8. Methylen-(6R, 7R)-7-[2-(2-furyl)-2-(methoxyimino)acetamido]-8-oxo-3-/[(1,4,5,6-tetrahydro-4-methyl-5,6-dioxo-as-triazin-3-yl)thio]methyl/-5-thia-1-azabicyclo[4.2.0]oct-2-en-2-carboxylat-pivalat (Z-Isomer), sowie Salze dieser Verbindung und Hydrate dieser Verbindung und von deren Salzen.

9. Verbindungen gemäss einem der Ansprüche 1-8 als pharmazeutische Wirkstoffe.

10. Verfahren zur Herstellung von leicht hydrolysierbaren Estern und Aethern von Cephalosporinen der allgemeinen Formel I gemäss Anspruch 1 sowie von Salzen dieser Verbindungen und von Hydraten dieser Ester, Aether und Salze, dadurch gekennzeichnet, dass man

a) eine Carbonsäure bzw. ein Enol der Formel I einer entsprechenden Veresterung bzw. Verätherung unterwirft oder dass man

b) einen leicht hydrolysierbaren Ester bzw. Aether einer Carbonsäure der allgemeinen Formel

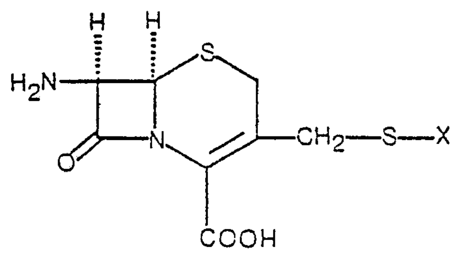

(II)

in der X die in Formel I gegebene Bedeutung hat mit einer Säure der allgemeinen Formel

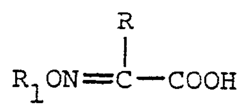

(III)

in der R und $R_1$ die in Formel I gegebene Bedeutung haben, oder mit einem reaktionsfähigen funktionellen Derivat dieser Säure umsetzt oder dass man

c) zur Herstellung eines leicht hydrolysierbaren Aethers einer Verbindung der Formel I eine Carbonsäure der allgemeinen Formel

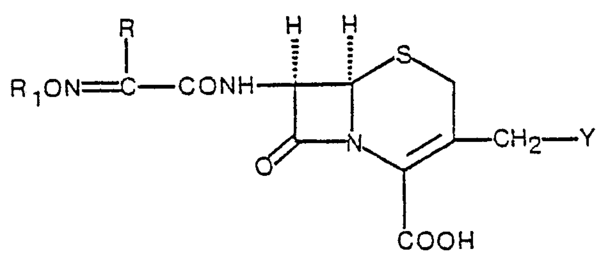

(IV)

in der R und $R_1$ die in Formel I gegebene Bedeutung haben und Y eine austretende Gruppe darstellt mit einem Thiol der allgemeinen Formel

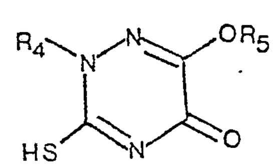

(V)

in der $R_4$ die in Formel I gegebene Bedeutung hat und $R_5$ eine leicht abspaltbare Aethergruppe darstellt, umsetzt, wonach man das Reaktionsprodukt gegebenenfalls in ein Salz oder Hydrat bzw. ein Hydrat dieses Salzes überführt.

11. Pharmazeutische Präparate, gekennzeichnet durch einen Gehalt an einer Verbindung gemäss einem der Ansprüche 1-8.

## Claims

1. Readily hydrolyzable esters and ethers of cephalosporins of the general formula

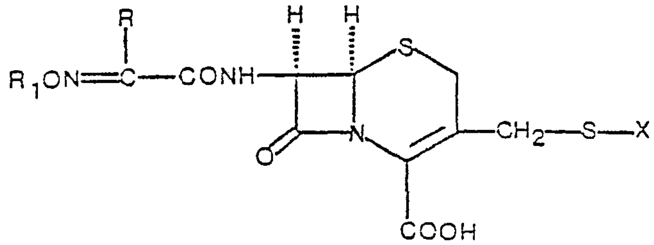

(I)

in which R represents furyl, $R_1$ represents $C_{1-7}$ alkyl and X represents a group of the formulae

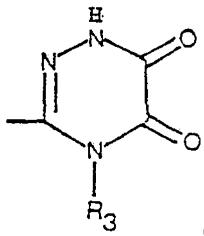

a

or

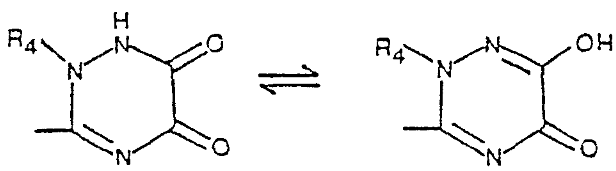

$c_1$ $c_2$

in which $R_3$ and $R_4$ represent $C_{1-7}$ alkyl, whereby «readily hydrolyzable esters» signifies compounds of formula I whose carboxy group is present in the form of a readily hydrolyzable ester group and «readily hydrolyzable ethers» signifies compounds of formula I with $X = c_2$ whose enolic OH group is present in the form of a readily hydrolyzable ether group, as well as of salts of these compounds and of hydrates of these esters, ethers and salts.

2. Esters and ethers according to claim 1, characterized in that they are $C_{1-7}$-alkyl-COO-$C_{1-7}$-alkyl esters or ethers.

3. Esters and ethers according to claim 2, characterized in that they are pivaloyloxymethyl esters or ethers.

4. Esters and ethers of cephalosporins of formula I according to any one of claims 1-3, characterized in that $R_1$ represents methyl, as well as salts of these compounds and hydrates of these esters, ethers and salts.

5. Esters and ethers of cephalosporins of formula I according to any one of claims 1-4, characterized in that X represents the 1,2,5,6-tetrahydro-2-methyl-5,6-dioxo-as-triazin-3-yl or the 2,5-dihydro-6-hydroxy-2-methyl-5-oxo-as-triazin-3-yl group, as well as salts of these compounds and hydrates of these esters, ethers and salts.

6. Esters and ethers of cephalosporins of formula I according to any one of claims 1-5, characterized in that X represents the 1,4,5,6-tetrahydro-4-methyl-5,6-dioxo-astriazin-3-yl group, as well as salts of these compounds and hydrates of these esters, ethers and salts.

7. Methylene (6R, 7R)-{/[2,5-dihydro-2-methyl-5-oxo-6-[(pivaloyloxy)methoxy]-as-triazin-3-yl]thio/methyl}-7-[2-(2-furyl)-2(methoxyimino)acetamido]-8-oxo-5-thia-1-azabicyclo[4.2.0]oct-2-ene-2-carboxylate pivalate (Z-isomer), as well as salts of this compound and hydrates of this compound and of its salts.

8. Methylene (6R, 7R)-7-[2-(2-furyl)-2-(methoxyimino)-acetamido]-8-oxo-3-/[(1,4,5,6-tetrahydro-4-methyl-5,6-dioxo-as-triazin-3-yl) thio]methyl/-5-thia-1-azabicyclo[4.2.0]oct-2-ene-2-carboxylate pivalate (Z isomer), as well as salts of this compound and hydrates of this compound and of its salts.

9. Compounds according to any one of claims 1-8 as pharmaceutically active substances.

10. Process for the manufacture of readily hydrolyzable esters and ethers of cephalosporins of general formula I according to claim 1 as well as of salts of these compounds and of hydrates of these esters, ethers and salts, characterized by

a) subjecting a carboxylic acid or an enol of formula I to a corresponding esterification or etherification, or

b) reacting a readily hydrolyzable ester or ether of a carboxylic acid of the general formula

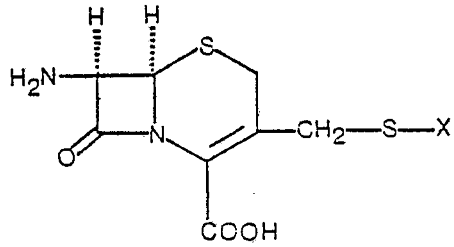

(II)

in which X has the significance given in formula I, with an acid of the general formula

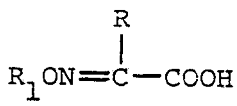

(III)

in which R and $R_1$ have the significance given in formula I, or with a reactive functional derivative of this acid, or

c) for the manufacture of a readily hydrolyzable ether of a compound of formula I, reacting a carboxylic acid of the general formula

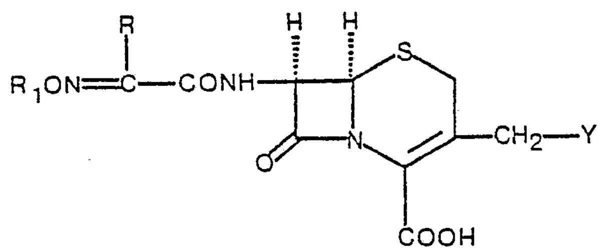

(IV)

in which R and $R_1$ have the significance given in formula I and Y represents a leaving group, with a thiol of the general formula

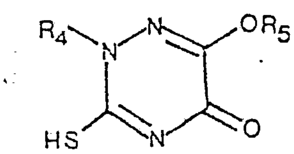

(V)

in which $R_4$ has the significance given in formula I and $R_5$ represents a readily cleavable ether group, whereafter, if desired, the reaction product is converted into a salt or hydrate or into a hydrate of this salt.

11. Pharmaceutical preparations, characterized by a content of a compound according to any one of claims 1-8.

**Revendications**

1. Esters et éthers facilement hydrolysables de céphalosporines de formule générale

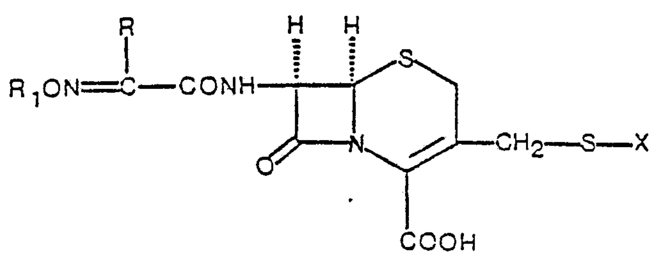

(I)

où R représente un furyle, $R_1$ un alcoyle en $C_1$ à $C_7$ et X un groupe de formules

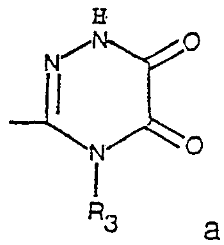

a

ou

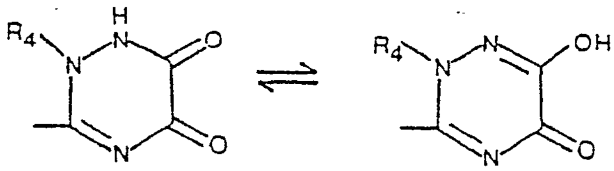

$c_1$ $c_2$

où $R_3$ et $R_4$ représentent un alcoyle en $C_1$ à $C_7$, où l'expression « esters facilement hydrolysables » représente des composés de formule I dont le groupe carboxy se présente sous la forme d'un groupe ester facilement hydrolysable et l'expression « éthers facilement hydrolysables » représente des composés de formule I où $X = c_2$, dont le groupe OH énolique se présente sous la forme d'un groupe éther facilement hydrolysable, ainsi que de sels de ces composés et des hydrates de ces esters, éthers et sels.

2. Esters et éthers selon la revendication 1, caractérisés en ce qu'ils représentent des alcoyles en $C_1$ à $C_7$-COO-alcoyle en $C_1$ à $C_7$-esters ou -éthers.

3. Esters et éthers selon la revendication 2, caractérisés en ce qu'ils représentent un pivaloyloxyméthylester ou -éther.

4. Esters et éthers de céphalosporines de formule I selon l'une des revendications 1-3, caractérisés en ce que $R_1$ représente un méthyle, ainsi que les sels de ces composés et les hydrates de ces esters, éthers et sels.

5. Esters et éthers de céphalosporines de formule I selon l'une des revendications 1-4, caractérisés en ce que X représente le groupe 1,2,5,6-tétrahydro-2-méthyl-5,6-dioxoas-triazin-3-yle ou le groupe 2,5-dihydro-6-hydroxy-2-méthyl-5-oxo-as-triazin-3-yle ainsi que les sels de ces composés et les hydrates de ces esters, éthers et sels.

6. Esters et éthers de céphalosporines de formule I selon l'une des revendications 1-5, caractérisés en ce que X représente le groupe 1,4,5,6-tétrahydro-4-méthyl-5,6-dioxo-as-triazin-3-yle, ainsi que les sels de ces composés et les hydrates de ces esters, éthers et sels.

7. Méthylène-(6R, 7R)-3-{/[2,5-dihydro-2-méthyl-5-oxo-6-[(pivaloyloxy)méthoxy]-as-triazin-3-yl]-thio/méthyl}-7-[2-(2-furyl)-2-(méthoxyimino)acétamido]-8-oxo-5-thia-1-azabicyclo[4.2.0]oct-2-ène-2-carboxylate-pivalate (isomère Z), ainsi que les sels de ce composé et les hydrates de ce composé et de ses sels.

8. Méthylène-(6R, 7R)-7-[2-(2-furyl)-2-(méthoxyimino)acétamido]-8-oxo-3-/[(1,4,5,6-tétrahydro-4-méthyl-5,6-dioxo-as-triazin-3-yl)thio]méthyl/-5-thia-1-azabicyclo[4.2.0]oct-2-ène-2-carboxylate-pivalate (isomère Z) ainsi que les sels de ce composé et les hydrates de ce composé et de ses sels.

9. Composés selon l'une des revendications 1-8 comme substances actives pharmaceutiques.

10. Procédé de préparation d'esters et éthers facilement hydrolysables de céphalosporines de formule générale I selon la revendication 1 ainsi que des sels de ces composés et d'hydrates de ces esters, éthers et sels, caractérisé en ce que

a) on soumet un acide carboxylique ou un énol de formule I à une estérification ou éthérification correspondante, ou

b) on fait réagir un ester ou éther facilement hydrolysable d'un acide carboxylique de formule générale

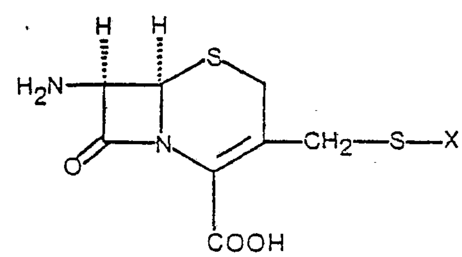

(II)

où X a la signification donnée dans la formule I avec un acide de formule générale

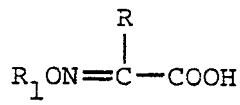

(III)

où R et $R_1$ ont la signification donnée dans la formule I, ou avec un dérivé fonctionnel réactif de cet acide, ou

c) pour préparer un ester facilement hydrolysable d'un composé de formule I on fait réagir un acide carboxylique de formule générale

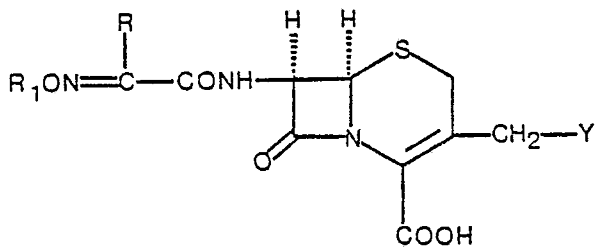

(IV)

où R et $R_1$ ont la signification donnée dans la formule I et où Y représente un groupe sortant, avec un thiol de formule générale

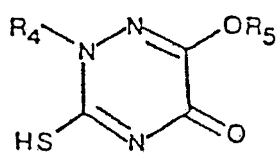

(V)

où $R_4$ a la signification donnée dans la formule I et où $R_5$ représente un groupe éther facilement détachable, puis on transforme le cas échéant le produit de la réaction en un sel ou un hydrate ou un hydrate de ce sel.

11. Préparations pharmaceutiques caractérisées en ce qu'elles contiennent un composé selon l'une des revendications 1-8.